# FASCICULE DE BREVET EUROPEEN

(11) **EP 2 535 460 B1**
(45) Date de publication et mention de la délivrance du brevet: **12.08.2015**
(21) Numéro de dépôt: 12360046.2
(22) Date de dépôt: 11.06.2012
(51) Int. Cl.: E02D 1/02, G01N 3/48, G01N 33/24, G01N 3/42, E02D 33/00

(54) **Dispositif de mesure de la résistance à la pénétration d'un sol**
Vorrichtung zur Messung des Eindringwiderstands von Böden
Device for measuring the resistance to ground penetration

(30) Priorité: 16.06.2011 FR 1155252
(43) Date de publication de la demande: 19.12.2012
(73) Titulaire: Hydro Geotechnique, 71150 Fontaines (FR)
(72) Inventeur: Gress, Jean-Baptiste, 21200 Levernois (FR)
(74) Mandataire: Koelbel, Caroline

(56) Documents cités:
- EP-A1- 0 544 606
- FR-A1- 2 584 186
- US-A- 3 958 646

## Description

### Domaine technique :

La présente invention concerne un dispositif de mesure de la résistance à la pénétration d'un sol, appelé communément un pénétromètre, et comportant au moins une tige de mesure terminée par une pointe de mesure, au moins un tube de protection extérieur disposé autour de ladite tige de mesure, des moyens de descente et de relevage permettant d'effectuer un enfoncement dans le sol en mode statique du couple formé par ladite tige de mesure et ledit tube de protection, des moyens de frappe permettant d'effectuer un enfoncement dans le sol en mode dynamique du couple formé par ladite tige de mesure et ledit tube de protection, et des moyens de mesure permettant de déterminer la résistance à la pénétration dudit sol.

### Technique antérieure :

De manière traditionnelle, les dispositifs de mesure de ce type sont utilisés en géotechnique pour sonder le sol et mesurer sa résistance à l'enfoncement, soit en mode statique, soit en mode dynamique, sur des profondeurs pouvant atteindre plusieurs dizaines de mètres, au moyen de tiges raccordées bout à bout pour former un train de tiges au bout duquel est fixée une pointe de mesure normalisée.

En mode statique, le train de tiges est poussé par des vérins et la pointe normalisée mesure électriquement ou mécaniquement la résistance de pointe (qc) et éventuellement le frottement latéral (qs) sur un manchon cylindrique situé au-dessus de la pointe au fond du sondage. Ces mesures sont enregistrées de manière continue ou discontinue selon un pas régulier. La mesure statique de la résistance à la pénétration du sol est sans conteste la plus précise, car elle est réalisée directement sur la pointe au fond du sondage. Par contre, l'utilisation de ce mode statique est limitée par la capacité de la machine (poids, puissance et qualité d'ancrage de la machine), le frottement exercé par le sol sur le train de tiges, et la résistance du sol. En conséquence, ce mode statique est plus particulièrement dédié aux sols de résistance faible à moyenne.

Le mode dynamique consiste à déduire la résistance à la pénétration du sol en comptabilisant le nombre de coups de mouton nécessaire à un enfoncement donné du train de tiges au bout duquel est fixé une pointe de section définie. Le battage est réalisé en faisant chuter une masse ou un marteau sur une enclume enfonçant le train de tiges par à-coups. Ce mode dynamique offre davantage de puissance et de capacité de pénétration du train de tiges dans un sol. De fait, son utilisation est dédiée aux sols de compacité faible à élevée.

Certains pénétromètres, tels que celui décrit dans la publication EP 0 544 606 A1, à l'arrêt du mode statique, enfoncent alors un train de tiges simple en mode dynamique sans effectuer de mesure, puis repassent en mode statique lorsque la compacité redevient plus faible, en mesurant alors un effort total de résistance en pointe (qc) et de frottement sur la surface latérale du train de tiges simple, dont le diamètre est constant. Ils mesurent ensuite l'effort d'arrachement de ce train de tiges, à pas réguliers, pour en déduire un frottement latéral (qs) à l'enfoncement, qu'ils déduisent de l'effort total pour obtenir une résistance en pointe seule. Cette approche est totalement erronée.

Lorsque la résistance des couches traversées est telle que le mode statique n'est plus possible, d'autres pénétromètres poursuivent la pénétration dynamique mais dissociée du train de tubes périphérique et du train de tiges axial par passes alternées. La mesure sur la pointe s'effectue alors à l'aide d'une masse définie chutant d'une hauteur donnée : c'est alors le nombre de coups de moutons qui détermine la résistance de pointe (qc) et du frottement latéral (qs). Ils ne permettent donc plus d'effectuer une mesure statique de la résistance de pointe (qc) et du frottement latéral (qs).

La publication FR-A-2 584 186 propose un pénétromètre dans lequel la tête de battage est rigidement solidaire du train de tiges creuses mais élastiquement solidaire du train de tiges pleines par l'interposition d'un ressort. En mode d'enfoncement dynamique, la chute du mouton de battage provoque l'enfoncement du train de tiges creuses qui bute sur la pointe de mesure ainsi entraînée et comprime le ressort. Or cette configuration ne permet pas de maîtriser la vitesse de pénétration dans le sol et provoque des effets parasites voire des phénomènes de résonance conduisant à des mesures de résistance du sol à la pointe aberrantes et non exploitables.

A ce jour, aucun dispositif de mesure ne permet de mesurer en mode statique le terme de pointe (qc) et le terme de frottement latéral (qs) quel que soit le mode d'enfoncement statique ou dynamique du train de tubes de protection et de tiges de mesure.

### Exposé de l'invention:

La présente invention propose une solution aux problèmes évoqués ci-dessus en fournissant un dispositif de mesure capable de travailler en mode mixte dynamique et statique dès que le mode tout statique n'est plus possible lorsque la résistance du sol devient trop importante, tout en conservant une mesure statique de la résistance en pointe réalisée au niveau de la pointe de mesure, complétée le cas échéant par une mesure du frottement latéral réalisée sur un manchon au-dessus de la pointe. On augmente ainsi la capacité de pénétration du train de mesures et on obtient des résultats de mesure précis, fiables et reproductibles.

Dans ce but, l'invention concerne un dispositif de mesure de la résistance à la pénétration d'un sol selon l'énoncé de la revendication indépendante 1.

Cette construction particulière permet d'isoler l'enfoncement dynamique du tube de protection extérieur, de la prise de mesure statique de la résistance en pointe et du frottement latéral à travers la tige de mesure intérieure. Ainsi, la tige de mesure ne subit pas les efforts mécaniques exercés par les moyens d'enfoncement dynamique et la prise de mesure à la pointe peut être réalisée en mode statique.

Dans la forme de réalisation préférée, les moyens de mesure comportent une jauge associée à un capteur de pression. Ce capteur de pression peut être hydraulique et la jauge peut être délimitée entre une partie fixe et une partie mobile dudit bloc de montage, la partie mobile étant agencée pour être couplée à la tige de mesure par des moyens de verrouillage.

Ces moyens de verrouillage peuvent comporter au moins un verrou couplé à un actionneur pour être mobile dans une direction perpendiculaire à l'arbre central entre une position libre dans laquelle la tige de mesure est découplée des moyens de mesure et une position verrouillée dans laquelle la tige de mesure est couplée aux moyens de mesure.

L'arbre central comporte, avantageusement, une fente axiale traversante pour autoriser le déplacement dudit verrou en position verrouillée et un évidement axial pour recevoir un prolongateur de ladite tige de mesure.

Un témoin peut être disposé à l'opposé de l'actionneur pour être visible de l'extérieur dudit bloc de montage par un opérateur, ce témoin coopérant avec le verrou pour occuper une position rentrée lorsque le verrou est en position libre et une position sortie lorsque le verrou est en position verrouillée.

L'arbre central est monté coulissant dans le bloc de montage sur une course contrôlée.

L'extrémité inférieure dudit arbre central peut comporter un embout de raccordement pour recevoir le tube de protection. Dans ce cas, un guide d'enfoncement peut être fixé audit embout de raccordement et peut être pourvu d'un logement agencé pour coiffer une tête d'enfoncement fixée sur le tube de protection.

Le dispositif de mesure peut également comporter une tête d'arrachage agencée pour être fixée audit embout de raccordement et pourvue d'un logement agencé pour coiffer une pièce d'extraction fixée au tube de protection, ainsi qu'une fourchette d'arrachage agencée pour s'emboîter dans la pièce d'extraction et solidariser ledit tube de protection audit arbre central.

### Description sommaire des dessins :

La présente invention et ses avantages apparaîtront mieux dans la description suivante d'un mode de réalisation donné à titre d'exemple non limitatif, en référence aux dessins annexés, dans lesquels:
- la figure 1 est une vue de face d'un dispositif de mesure selon l'invention,
- la figure 2 est une vue en coupe axiale du détail II du dispositif de la figure 1,
- la figure 3 est une vue en coupe axiale selon l'axe III-III du dispositif de la figure 2 équipé d'une pointe de mesure simple,
- la figure 4 est une vue similaire à la figure 3 du dispositif équipé d'une pointe de mesure double, et
- la figure 5 est une vue en coupe axiale selon l'axe III-III du dispositif de la figure 2 équipé d'une tête d'arrachage permettant l'extraction des trains de tiges du sol.

### Illustrations de l'invention et meilleure manière de la réaliser:

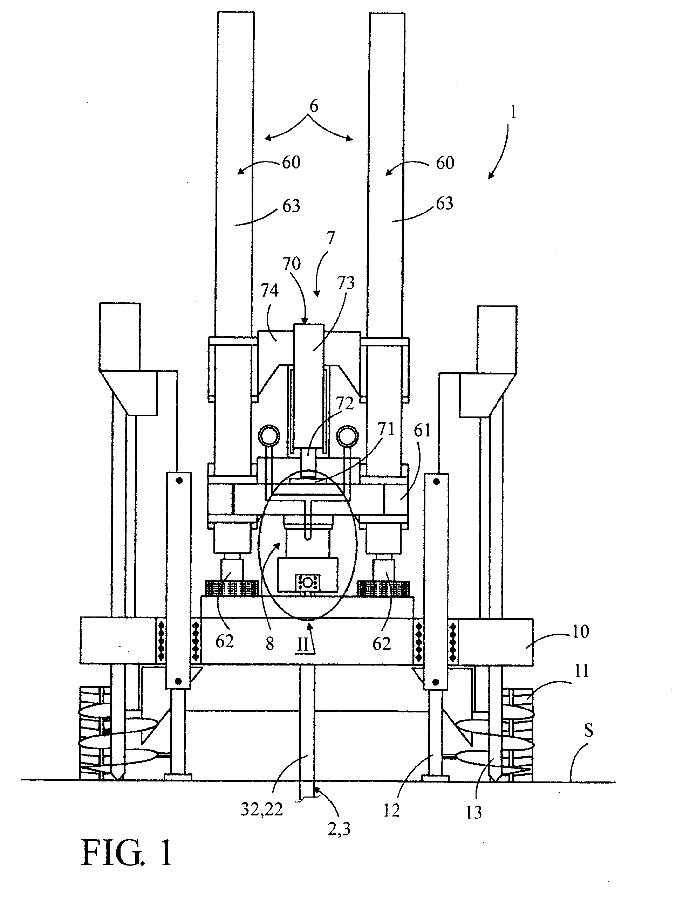

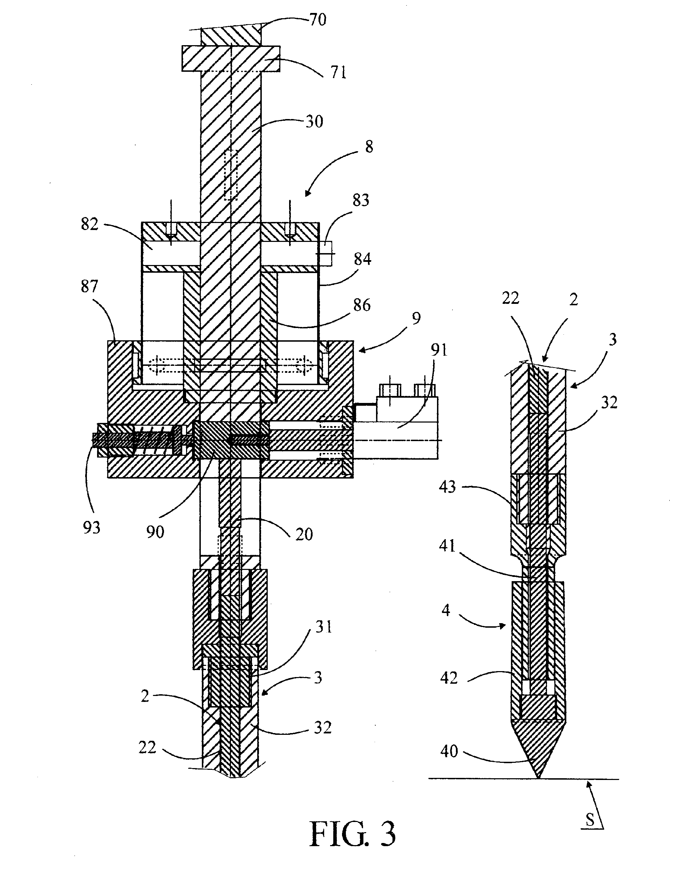

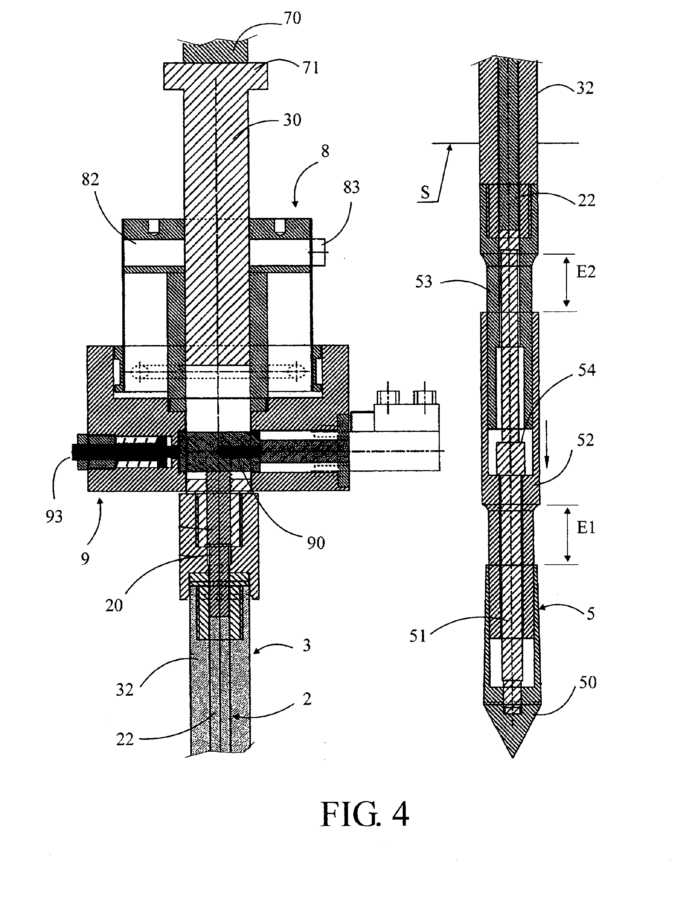

En référence à la figure 1, le dispositif de mesure 1 de la compacité d'un sol selon l'invention comporte un châssis 10 porté par des chenilles 11, des roues ou similaires, ou encore porté par un camion, une remorque ou similaire. Ce châssis 10 est équipé de pieds 12 et de tarières d'ancrage 13 pour stabiliser et ancrer le dispositif de mesure 1 au sol S. Toute autre configuration peut être envisagée, l'essentiel étant d'assurer les fonctions de stabilisation et d'ancrage du dispositif de manière efficace. Le dispositif de mesure 1 comporte au moins une tige de mesure 22 terminée par une pointe de mesure simple 4 ou double 5 (cf. Figure 3 et 4) et agencée pour sonder le sol S grâce à des moyens de descente et de relevage 6 et des moyens de frappe 7. Il comporte également des moyens de mesure permettant de déterminer la résistance à la pénétration du sol S au fur et à mesure de l'enfoncement de la tige de mesure 22 dans les différentes couches de sol traversées, par une mesure de la résistance de pointe (qc) complétée le cas échéant par une mesure du frottement latéral (qs) sur un manchon associé à la pointe de mesure.

Les moyens de descente et de relevage 6 comportent au moins un et dans l'exemple illustré deux vérins 60 embarqués sur une poutre mobile 61 portant un arbre central 30 par l'intermédiaire d'un bloc de montage 8. Dans l'exemple illustré, la tige 62 des vérins 60 est solidaire du châssis 10 et la chemise 63 des vérins 60 est solidaire de la poutre mobile 61. La configuration inverse est également possible. De même, le nombre de vérins 60 n'est pas limité à deux et pourra dépendre de la puissance d'enfoncement à développer.

Les moyens de frappe 7 comportent dans l'exemple illustré un marteau 70 embarqué sur les vérins 60 et agissant sur une enclume 71 solidaire de l'extrémité supérieure de l'arbre central 30. La chemise 73 du marteau 70 est solidaire des chemises 63 des vérins 60 par une potence 74 (cf. figure 1) et la pointerolle 72 du marteau 70 est disposée à l'aplomb de l'enclume 71. Les vérins 60 et le marteau 70 sont commandés de préférence par une centrale hydraulique, présente sur tous les engins de chantier. Mais toute autre source d'énergie est envisageable.

Le dispositif de mesure 1 de l'invention se distingue de l'état de l'art en ce qu'il comporte au moins un tube de protection 32 extérieur disposé autour de la tige de mesure 22 portant la pointe de mesure 4, 5, moyennant un jeu autorisant un coulissement libre de la tige de mesure 22 par rapport au tube de protection 32 et inversement. En référence également aux autres figures, la tige de mesure 22 est agencée pour être couplée aux moyens de mesure par des moyens de verrouillage 9 expliqués plus loin et le tube de protection 32 est agencée pour être couplé aux moyens de descente et de relevage 6 ainsi qu'aux moyens de frappe 7 par l'arbre central 30. Ainsi la partie « enfoncement dynamique » est découplée de la partie « prise de mesure ». Bien entendu, au fur et à mesure de l'enfoncement dans le sol, la tige de mesure 22 est prolongée par d'autres tiges de mesure formant un train de tiges de mesure 2 intérieures et concentriques, et le tube de protection 32 est prolongé par d'autres tubes de protection formant un train de tubes de protection 3 extérieurs et concentriques.

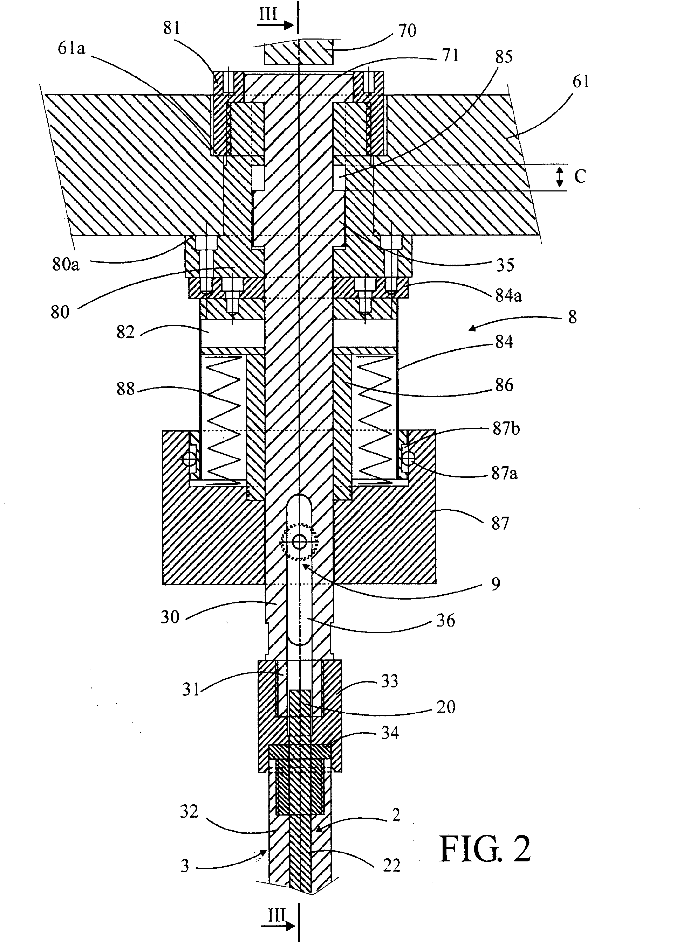

En référence plus particulièrement à la figure 2, l'arbre central 30 monté coulissant dans le bloc de montage 8 et pourvu d'une extrémité supérieure formant l'enclume 71 et d'une extrémité inférieure formant un embout de raccordement 31 pour recevoir le tube de protection 32. Un guide d'enfoncement 33 est fixé sur l'embout de raccordement 31 par vissage et comporte un logement agencé pour coiffer une tête d'enfoncement 34 fixée sur le tube de protection 32 par vissage. Bien entendu, tout autre moyen d'assemblage et/ou de fixation équivalent est envisageable, l'essentiel étant que l'effort d'enfoncement exercé axialement par l'arbre central 30 puisse être transmis axialement sur le tube de protection 32, et qu'il soit possible d'ajouter facilement des couples « tube de protection 32 extérieur - tige de mesure 22 intérieure » au fur et à mesure de l'enfoncement dans le sol.

L'arbre central 30 est agencé pour pouvoir se déplacer par rapport à la poutre mobile 61 sur une course déterminée. Cette course est notamment délimitée par des nervures 35 qui s'étendent radialement de l'arbre central 30 et qui sont logées dans des rainures 85 de section complémentaire prévues dans le bloc de montage 8, la hauteur des rainures 85 étant supérieure à celle des nervures 35 autorisant un débattement libre sur une course "C". L'ouverture supérieure du bloc de montage 8 comporte une section complémentaire à celle de l'arbre central 30 au droit des nervures 35 pour pouvoir monter l'arbre central 30 à l'intérieur du bloc de montage 8 à la manière d'une baïonnette.

Le bloc de montage 8 comporte un corps central 80 logé dans un alésage traversant de la poutre mobile 61. Il est fixé à la poutre mobile 61 d'un côté par son épaulement 80a en appui sur la face inférieure de la poutre et de l'autre côté par une pièce écrou 81 vissée jusqu'en butée contre un épaulement 61a de la poutre. La pièce écrou 81 est creuse pour le passage de l'arbre central 30 et permettre de placer l'enclume 71 au droit du marteau 70.

Ce bloc de montage 8 comporte les moyens de mesure de la résistance de pointe (qc) à la pénétration du sol et du frottement latéral (qs) sous la forme d'une jauge 82 associée à un capteur de pression 83. Dans l'exemple illustré, le capteur de pression 83 est hydraulique et la jauge 82 est délimitée entre une partie fixe 84 et une partie mobile 86 du bloc de montage 8 couplée à la tige de mesure 22. La partie fixe constitue une chemise 84 d'un vérin à simple effet et est fixée à la base du corps central 80 par une plaque de montage 84a et des organes de vissage. La partie mobile constitue un piston 86 d'un vérin à simple effet et est couplée à la tige de mesure 22 par des moyens de verrouillage 9 expliqués plus loin. Le piston 86 est suspendu au bloc central 80 par l'intermédiaire d'un support 87 couplé à la chemise 84 par des tiges de liaison 87a disposées dans des logements tangentiels 87b ménagés entre le bloc de support 87 et la chemise 84. Les tiges de liaison 87a permettent de bloquer en rotation le support 87 et les logements tangentiels 87b permettent de délimiter la course axiale du piston 86. Ce piston 86 est sollicité dans une direction opposée à la jauge 82 par des ressorts de traction 88 prévus dans la chemise 84. Bien entendu, tout autre moyen de mesure de pression est compatible avec le dispositif de l'invention, tel que par exemple un capteur de pression électrique ou électronique, de forme annulaire pour le passage de l'arbre central 30.

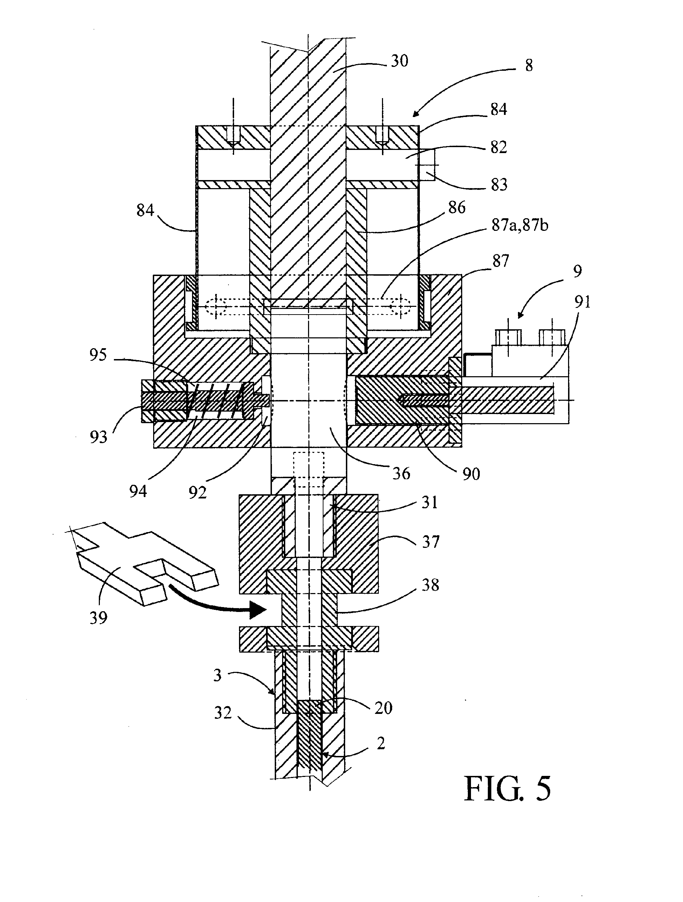

Les moyens de verrouillage 9 sont mieux représentés à la figure 5 et sont agencés pour coupler la tige de mesure 22 au support 87 dans le but d'enregistrer la résistance du sol à la pénétration par la détection d'une augmentation de pression due à la réduction du volume de la jauge 82. Ils comportent un verrou 90 couplé à un actionneur 91 pour être mobile transversalement entre une position libre dans laquelle la tige de mesure 22 est libre par rapport au support 87 (cf. fig. 2 et 5), et une position verrouillée dans laquelle la tige de mesure 22 est couplée au support 87 (cf. fig. 3 et 4). L'actionneur 91 peut être un vérin raccordé sur la centrale hydraulique des autres vérins 60, 70, ou tout autre type d'actionneur linéaire. Le support 87 est perforé de part en part suivant un axe transversal coupant perpendiculairement l'axe de l'arbre central 30. Il définit un premier alésage de guidage 92 qui s'étend au delà de l'arbre central 30 et dans lequel coulisse le verrou 90 sous l'effet du vérin 91 entre ses deux positions. En correspondance, l'arbre central 30 comporte une fente axiale 36 autorisant le passage du verrou 90 en position bloquée et s'étendant sur une hauteur déterminée autorisant un débattement axial de l'arbre central 30 par rapport au verrou 90. Il comporte également un évidement axial débouchant dans cette fente axiale 36 pour recevoir le prolongateur 20 de la tige de mesure 22 en contact avec ledit verrou 90 pour actionner la jauge 82.

A l'opposé du vérin 91 s'étend un témoin 93 débouchant à l'extérieur du support 87 pour être visible de l'extérieur par un opérateur. Le témoin 93 est logé dans un second alésage de guidage 94 ménagé dans le support 87 en prolongement du premier alésage de guidage 92. Il comporte une extrémité qui débouche dans le premier alésage de guidage 92 et vers lequel il est assujetti par un ressort de compression 95. Ce témoin 93 est agencé pour coopérer avec le verrou 90 pour occuper une position rentrée lorsqu'il est en position libre (cf. fig. 2 et 5) et une position sortie lorsqu'il est en position verrouillée (cf. fig. 3 et 4) afin de renseigner l'opérateur si la prise de mesure est activée ou non.

En référence à la figure 5, le dispositif de mesure 1 comporte en outre des moyens d'extraction permettant d'extraire du sol les trains de tiges 2 et 3 après sondage. Ils sont pourvus d'une tête d'arrachage 37 agencée pour être fixée par vissage à l'embout de raccordement 31 de l'arbre central 30, cette tête étant pourvue d'un logement agencé pour coiffer une pièce d'extraction 38 fixée par vissage au tube de protection 32. Ils comportent également une fourchette d'arrachage 39 agencée pour s'emboîter dans la pièce d'extraction 38 et ainsi solidariser les trains de tiges 2 et 3 à la poutre mobile 61 par l'intermédiaire de l'arbre central 30 pour permettre le relevage des trains de tiges 2 et 3 par les vérins 60. Bien entendu, tout autre moyen permettant l'extraction des trains de tiges est compatible.

### Possibilités d'application industrielle :

Le dispositif de mesure peut être équipé d'une tête de mesure simple 4 (cf. figure 3) ou d'une tête de mesure double 5 (cf. figure 4) selon la nature du sol sondé et les mesures à réaliser. La figure 3 montre l'utilisation d'une tête de mesure simple 4 à l'extrémité du train de tiges de mesure 2 entouré du train de tubes de protection 3. La pointe de mesure 4 comporte une tête conique 40 portée par une tige 41 prolongeant les tiges de mesure 22. Cette pointe de mesure 4 est protégée par un manchon 42 monté coulissant sur une pièce de montage 43 fixée par vissage à l'extrémité du premier tube de protection 32. Pour pouvoir monter le premier couple « tige de mesure 22 et tube de protection 32 » sur l'embout de raccordement 31 de l'arbre central 30, la poutre mobile 61 est relevée en position haute par les vérins 60 et les moyens de verrouillage 9 sont placés en position libre permettant la remontée de l'arbre central 30 sur la course C. Après montage, les vérins 60 sont actionnés pour descendre la poutre mobile 61 selon un mouvement continu. Le marteau 70 étant en contact avec l'enclume 71 transmet ce mouvement de descente à l'arbre central 30 puis au tube de protection 32 qui s'enfonce dans le sol en emportant avec lui la tige de mesure 22 qui peut descendre d'elle même dans le sol par gravité en coulissant à l'intérieur du tube de protection 32. Préalablement, les moyens de verrouillage 9 sont activés en position verrouillée pour mettre en contact le verrou 90 avec le prolongateur 20 de la tige de mesure 22, actionner la jauge 82 et permettre ainsi la prise de mesure par la tige de mesure 22. Le dispositif de mesure 1 fonctionne en mode statique et en mode continu. Dans cette configuration, les moyens de mesure sont mis en série avec la tige de mesure 22 et la résistance à la pénétration exercée par le sol sur la pointe de mesure 4, correspondant à la résistance de pointe (qc), peut être détectée par le capteur de pression 83 couplé à la jauge 82 et enregistrée de manière continue ou discontinue. Bien entendu, des moyens d'acquisition de données automatiques peuvent être connectés au capteur de pression 83.

Si au cours du sondage, la résistance à la pénétration du sol augmente et que l'enfoncement n'est plus possible en mode statique, alors les moyens de frappe 7 sont activés pour passer en mode dynamique. Le marteau 70 frappe sur l'enclume 71 par à-coups pour créer des vibrations sur le tube de protection 32 et dépasser la résistance au frottement du sol. En cours de frappe, les efforts de frappe du marteau 70 sont transmis directement de l'arbre central 30 au tube de protection 32 sans passer par la tige de mesure 22 (cf. fig. 2) permettant ainsi de ne pas transmettre l'énergie dynamique à la pointe de mesure 4. Grâce à cette cinématique, il est toujours possible d'enregistrer la résistance du sol à la pointe (qc) d'où des résultats de mesure précis et représentatifs de la réalité car les moyens de mesure sont maintenus en série avec la tige de mesure 22 par l'intermédiaire des moyens de verrouillage 9.

La figure 4 illustre l'utilisation d'une pointe de mesure double 5 permettant d'effectuer des mesures en mode discontinu, aussi bien en mode statique qu'en mode dynamique. Cette pointe de mesure 5 comporte une tête conique 50 portée par une tige 51 prolongeant les tiges de mesure 22. Un manchon de frottement 52 est pourvu d'un tronçon inférieur sur lequel la tête conique 50 peut coulisser et d'un tronçon supérieur monté coulissant sur une pièce de montage 53 fixée par vissage à l'extrémité du premier tube de protection 32. En mode discontinu, la mesure s'effectue par paliers, à savoir une étape d'enfoncement de l'ensemble « tube de protection 32 et tige de mesure 22 » à une profondeur déterminée suivi d'une étape de retrait et d'une phase de mesure sur une course déterminée correspondant au déploiement de la pointe de mesure 5. Cette technique permet d'effectuer deux mesures :
- la résistance de pointe (qc),
- la résistance cumulée de pointe (qc) et de frottement latéral (qs) sur le manchon permettant d'obtenir « qc+qs ».

Phase 1 : lors de l'étape d'enfoncement du tube de protection 32 et de la tige de mesure 22, le verrou 90 est en position déverrouillée et les moyens de mesure sont désactivés. La pointe de mesure double 5 (fig. 4) peut ainsi librement se contracter sous l'effet de l'enfoncement. Il n'y a pas de prise de mesure.

Phase 2 : à la profondeur souhaitée de l'essai, l'ensemble « vérin 60, marteau 70, tube de protection 32 et tige de mesure 22 » est remonté d'une hauteur suffisante pour permettre le verrouillage du verrou 90 à l'aide du vérin 9 et la mise en série des moyens de mesure avec la tige de mesure 22.

Phase 3 : durant la phase suivante, l'ensemble « vérin 60, marteau 70. tube de protection 32 et tige de mesure 22 » est redescendu sur la longueur de déplacement total « E1+E2 » de la pointe de mesure double 5 (7 cm par exemple). Sur les premiers centimètres, correspondant à la course E1 (3,5 cm par exemple), la tige de mesure 22 exerce un effort sur la tête conique 50 uniquement. La conception de la pointe de mesure double 5, à travers l'existence d'une butée 54, engendre automatiquement, lors de la poursuite de l'enfoncement, le déploiement de la tête conique 50 et du manchon de frottement 52 sur la seconde course E2 (par exemple 3,5 cm).

Lors de la première partie (E1) de déploiement de la pointe de mesure double 5, la contrainte mesurée par la jauge 82 correspond à la valeur de résistance en pointe « qc » alors que lors de la deuxième partie (E2) de déploiement, la contrainte mesurée correspond à la valeur de résistance en pointe et de frottement latéral « qc+qs ».

La valeur de « qs » est déduite simplement par différence entre les deux valeurs de contrainte mesurées par la jauge 82 pendant le déploiement complet de la pointe de mesure double 5.

Il ressort clairement de cette description que l'invention permet d'atteindre les buts fixés, à savoir mesurer directement et réellement l'effort nécessaire au déploiement de la pointe de mesure suivant le mode statique quel que soit le mode d'enfoncement statique ou dynamique du train de mesures. La présente invention n'est pas limitée à l'exemple de réalisation décrit mais s'étend à toute modification et variante évidentes pour un homme du métier tout en restant dans l'étendue de la protection définie dans les revendications annexées.

## Revendications

1. Dispositif de mesure (1) de la résistance à la pénétration d'un sol, appelé communément un pénétromètre, et comportant au moins une tige de mesure (22) terminée par une pointe de mesure (4, 5), au moins un tube de protection (32) extérieur disposé autour de ladite tige de mesure (22), des moyens de descente et de relevage (6) permettant d'effectuer un enfoncement dans le sol en mode statique du couple formé par ledit tube de protection (32) et ladite tige de mesure (22), des moyens de frappe (7) permettant d'effectuer un enfoncement dans le sol en mode dynamique du couple formé par ledit tube de protection (32) et ladite tige de mesure (22), et des moyens de mesure permettant de déterminer la résistance à la pénétration dudit sol, le dispositif (1) étant **caractérisé en ce que** :
- ledit tube de protection (32) est disposé autour de ladite tige de mesure (22) moyennant un jeu autorisant un coulissement libre de la tige de mesure par rapport au tube de protection et inversement,
- lesdits moyens de descente et de relevage (6) comportent au moins un actionneur (60) agencé pour déplacer en translation verticale une poutre mobile (61) dans laquelle est monté un arbre central (30) agencé pour transférer l'effort de descente et de relevage audit tube de protection (32), ledit arbre central (30) pouvant se déplacer par rapport à la poutre mobile (61) sur une course déterminée (C),
- lesdits moyens de frappe (7) sont embarqués sur ladite poutre mobile (61) et comportent au moins un marteau (70) agencé pour frapper une enclume (71) solidaire dudit arbre central (30) agencé pour transférer l'effort de frappe audit tube de protection (32) de sorte que les efforts de frappe en mode dynamique sont transmis directement audit tube de protection (32) sans passer par ladite tige de mesure (22),
- ledit arbre central (30) est monté dans ladite poutre mobile (61) par l'intermédiaire d'un bloc de montage (8) qui comporte lesdits moyens de mesure et dans lequel l'arbre central (30) est monté coulissant, ledit bloc de montage (8) comportant une chambre de compression (82) délimitée entre une partie fixe (84) et une partie mobile (86), et
- ledit arbre central (30) comporte un évidement axial pour recevoir en partie ladite tige de mesure (22) qui est couplée à ladite partie mobile (86) par des moyens de verrouillage (9), lesdits moyens de verrouillage (9) ayant deux positions, l'une dite verrouillée où la tige de mesure (22) est couplée à la partie mobile (86) ce qui permet de mesurer en mode statique au moins le terme de pointe (qc) quel que soit le mode d'enfoncement statique ou dynamique, et l'autre dite libre où la tige de mesure (22) est découplée de ladite partie mobile (86).

2. Dispositif (1)
selon la revendication 1, **caractérisé en ce que** lesdits moyens de mesure comportent au moins un capteur de pression (83) associé à une jauge (82).

3. Dispositif (1)
selon la revendication 2, **caractérisé en ce que** ledit capteur de pression (83) est hydraulique et **en ce que** ladite jauge (82) comporte une chambre de compression (82) délimitée entre une partie fixe (84) et une partie mobile (86) dudit bloc de montage (8), ladite partie mobile (86) étant agencée pour être couplée à ladite tige de mesure (22) par lesdits moyens de verrouillage (9).

4. Dispositif (1)
selon la revendication 3, **caractérisé en ce que** lesdits moyens de verrouillage (9) comportent au moins un verrou (90) couplé à un actionneur (91) pour être mobile dans une direction perpendiculaire à l'arbre central (30) entre une position libre dans laquelle ladite tige de mesure (22) est découplée desdits moyens de mesure et une position verrouillée dans laquelle ladite tige de mesure (22) est couplée auxdits moyens de mesure.

5. Dispositif (1)
selon la revendication 4, **caractérisé en ce que** ledit arbre central (30) comporte une fente axiale (36) traversante pour autoriser le déplacement dudit verrou (90) en position verrouillée et **en ce que** ledit évidement axial débouche dans cette fente axiale (36) pour recevoir un prolongateur (20) de ladite tige de mesure (22).

6. Dispositif (1)
selon la revendication 4, **caractérisé en ce qu'**un témoin (93) est disposé à l'opposé dudit actionneur (91) pour être visible de l'extérieur dudit bloc de montage (8) par un opérateur, ledit témoin (93) coopérant avec ledit verrou (90) pour occuper une position rentrée lorsque le verrou (90) est en position libre et une position sortie lorsque le verrou (90) est en position verrouillée.

7. Dispositif (1)
selon l'une quelconque des revendications précédentes, **caractérisé en ce que** ledit arbre central (30) est monté coulissant dans ledit bloc de montage (8) sur une course contrôlée.

8. Dispositif (1)
selon l'une quelconque des revendications précédentes, **caractérisé en ce que** l'extrémité inférieure dudit arbre central (30) comporte un embout de raccordement (31) pour recevoir ledit tube de protection (32).

9. Dispositif (1)
selon la revendication 8, **caractérisé en ce qu'**il comporte un guide d'enfoncement (33) agencé pour être fixé audit embout de raccordement (31) de l'arbre central (30), ce guide étant pourvu d'un logement agencé pour coiffer une tête d'enfoncement (34) fixée sur ledit tube de protection (32).

10. Dispositif (1)
selon la revendication 8, **caractérisé en ce qu'**il comporte une tête d'arrachage (37) agencée pour être fixée audit embout de raccordement (31) de l'arbre central (30), cette tête étant pourvue d'un logement agencé pour coiffer une pièce d'extraction (38) fixée dudit tube de protection (32), et **en ce qu'**il comporte une fourchette d'arrachage (39) agencée pour s'emboîter dans ladite pièce d'extraction (38) et solidariser ledit tube de protection (32) audit arbre central (30).

## Patentansprüche

1. Vorrichtung (1) zur Messung des Eindungwiderstands von Böden, gemeinhin als Penetrometer bezeichnet, bestehend aus zumindest einer Mess-Stange (22) mit einer Mess-Spitze (4, 5) an ihrem Ende, zumindest einem um besagte Mess-Stange (22) angeordneten externen Schutzrohr (32), Mittel zum Herunter- und Hochfahren (6), die es ermöglichen, das von besagtem Schutzrohr (32) und besagter Mess-Stange (22) gebildete Paar statisch in den Boden zu treiben, Schlagmittel (7), die es ermöglichen, das von besagtem Schutzrohr (32) und besagter Mess-Stange (22) gebildete Paar dynamisch in den Boden zu treiben, und Messmittel, die es ermöglichen, den Eindringwiderstand von besagtem Boden festzulegen, wobei die Vorrichtung **dadurch gekennzeichnet ist, dass**:
- besagtes Schutzrohr (32) um besagte Mess-Stange (22) mit einem Spiel angeordnet ist, das das freie Gleiten der Mess-Stange in Bezug auf das Schutzrohr und umgekehrt ermöglicht,
- besagte Mittel zum Herunter- und Hochfahren (6) zumindest einen Aktor (60) betragen, der ausgelegt ist, um einen beweglichen Balken (61) vertikal zu bewegen, in dem ein zentraler Schaft (30) montiert ist, der ausgelegt ist, um die Eintreib- und Hochfahrkraft auf besagtes Schutzrohr (32) zu übertragen,
- besagter zentraler Schaft (30) sich in Bezug auf den beweglichen Balken (61) auf einem bestimmten Hub (C) bewegen kann,
- besagte Schlagmittel (7) auf besagtem beweglichen Balken (61) angeordnet sind und zumindest einen Hammer (70) betragen, der ausgelegt ist, um auf einen Amboss (71) zu klopfen, der an besagtem zentralen Schaft (30) befestigt ist, wobei letzterer ausgelegt ist, um die Schlagkraft auf besagtes Schutzrohr (32) zu übertragen, so dass die dynamischen Schlagkräfte direkt auf besagtes Schutzrohr (32) übertragen werden, ohne über besagte Mess-Stange (22) zu gehen,
- besagter zentraler Schaft (30) in besagtem beweglichen Balken (61) über einen Anbaublock (8) montiert ist, der besagte Messmittel beträgt und in dem der zentrale Schaft (30) gleitend montiert ist, wobei besagter Anbaublock (8) eine zwischen einem festen Teil (84) und einem beweglichen Teil (86) begrenzte Druckkammer (82) beträgt, und
- besagter zentraler Schaft (30) eine axiale Aussparung aufweist, um besagte Mess-Stange (22) zum Teil aufzunehmen, die über Verriegelungs-Mittel (9) mit besagtem beweglichem Teil (86) gekoppelt ist, wobei besagte Verriegelungs-Mittel (9) zwei Stellungen aufweisen, eine sogenannte verriegelte Stellung, in der die Mess-Stange (22) mit dem beweglichem Teil (86) gekoppelt ist, wodurch zumindest der Spitzenwiderstand (qc) statisch gemessen werden kann, gleich ob bei statischem oder dynamischem Eintreiben, und die andere sogenannte freie Stellung, in der die Mess-Stange (22) vom besagtem beweglichen Teil (86) entkoppelt ist.

2. Vorrichtung (1) nach Anspruch 1, **dadurch gekennzeichnet, dass** besagte Messmittel zumindest einen mit einem Messgerät (82) zusammen arbeitenden Drucksensor (83) betragen.

3. Vorrichtung (1) nach Anspruch 2, **dadurch gekennzeichnet, dass** besagter Drucksensor (83) hydraulisch ist, und dadurch, dass besagtes Messgerät (82) eine zwischen einem festen Teil (84) und einem beweglichen Teil (86) von besagtem Anbaublock (8) begrenzte Druckkammer (82) beträgt, wobei besagter beweglicher Teil (86) ausgelegt ist, um durch besagte Verriegelungs-Mittel (9) an besagte Mess-Stange (22) gekoppelt zu werden.

4. Vorrichtung (1) nach Anspruch 3, **dadurch gekennzeichnet, dass** besagte Verriegelungs-Mittel (9) zumindest einen Riegel (90) betragen, der an einen Aktor (91) gekoppelt ist, um in einer zum zentralen Schaft (30) senkrechten Richtung beweglich zu sein, zwischen einer freien Stellung, in der besagte Mess-Stange (22) von besagten Messmitteln entkoppelt ist, und einer verriegelten Stellung, in der besagte Mess-Stange (22) an besagte Messmittel gekoppelt ist.

5. Vorrichtung (1) nach Anspruch 4, **dadurch gekennzeichnet, dass** besagter zentrale Schaft (30) einen durchgehenden axialen Schlitz (36) beträgt, um die Bewegung von besagtem Riegel (90) in seine verriegelte Stellung zu ermöglichen, und dadurch, dass besagte axiale Aussparung in diesem axialen Schlitz (36) mündet, um eine Verlängerung (20) von besagter Mess-Stange (22) aufzunehmen.

6. Vorrichtung (1) nach Anspruch 4, **dadurch gekennzeichnet, dass** ein Anzeiger (93) auf der besagtem Aktor (91) entgegen gesetzten Seite angeordnet ist, um außerhalb von besagtem Anbaublock (8) für einen Bediener sichtbar zu sein, wobei besagter Anzeiger (93) mit besagtem Riegel (90) zusammen arbeitet, um in einer eingezogenen Stellung zu sein, wenn Riegel (90) in seiner freien Stellung ist, und in einer herausragenden Stellung zu sein, wenn Riegel (90) in seiner verriegelten Stellung ist.

7. Vorrichtung (1) nach einem beliebigen der vorhergehenden Ansprüche, **dadurch gekennzeichnet, dass** besagter zentraler Schaft (30) auf einem kontrollierten Hub gleitend in besagtem Anbaublock (8) montiert ist.

8. Vorrichtung (1) nach einem beliebigen der vorhergehenden Ansprüche, **dadurch gekennzeichnet, dass** das untere Ende von besagtem zentralen Schaft (30) ein Anschlussstück (31) beträgt, um besagtes Schutzrohr (32) aufzunehmen.

9. Vorrichtung (1) nach Anspruch 8, **dadurch gekennzeichnet, dass** sie eine Eintreib-Führung (33) beträgt, die ausgelegt ist, um an besagtem Anschlussstück (31) des zentralen Schafts (30) befestigt zu werden, wobei diese Führung eine Aufnahme beträgt, die ausgelegt ist, um einen an besagtem Schutzrohr (32) befestigten Eintreibkopf (34) zu bedecken.

10. Vorrichtung (1) nach Anspruch 8, **dadurch gekennzeichnet, dass** sie einen Ausziehkopf (37) beträgt, der ausgelegt ist, um am besagtem Anschlussstück (31) des zentralen Schafts (30) befestigt zu werden, wobei dieser Kopf eine Aufnahme beträgt, die ausgelegt ist, um ein an besagtem Schutzrohr (32) befestigtes Abzugsteil (38) zu bedecken, und dadurch, dass sie eine Ausziehgabel (39) beträgt, die ausgelegt ist, um in besagtes Abzugsteil (38) eingefügt zu werden und besagtes Schutzrohr (32) an besagtem zentralen Schaft (30) zu befestigen.

## Claims

1. Device (1) for measuring the resistance to ground penetration, commonly called a penetrometer, and comprising at least one measuring rod (22) having a measuring point (4, 5) at its end, at least one outer protective tube (32) arranged around said measuring rod (22), down-feed and lifting means (6) that allow driving the couple formed by said protective tube (32) and said measuring rod (22) in the ground in static mode, percussion means (7) that allow driving the couple formed by said protective tube (32) and said measuring rod (22) in the ground in dynamic mode, and measuring means that allow determining the penetration resistance of said ground, the device being **characterized in that**:
- said protective tube (32) is arranged around said measuring rod (22) with a clearance that allows for a free sliding of the measuring rod with respect to the protective tube and vice-versa,
- said down-feed and lifting means (6) comprise at least one actuator (60) arranged to move in vertical translation a mobile beam (61) wherein a central shaft (30) is mounted, arranged to transfer the down-feed and lifting effort to said protective tube (32),
- said central shaft (30) can move with respect to said mobile beam (61) on a determined stroke (C),
- said percussion means (7) are loaded on said mobile beam (61) and comprise at least one hammer (70) arranged to hit an anvil (71) integral with said central shaft (30) arranged to transfer the percussion effort to said protective tube (32) so that the percussion efforts in dynamic mode are transmitted directly to said protective tube (32) without passing through said measuring rod (22),
- said central shaft (30) is mounted in said mobile beam (61) through a mounting block (8) that comprises said measuring means and wherein the central shaft (30) is slidingly mounted, said mounting block (8) comprising a compression chamber (82) delimited between a fixed section (84) and a mobile section (86), and
- said central shaft (30) comprises an axial recess to receive partly said measuring rod (22) that is coupled to said mobile section (86) by locking means (9), said locking means (9) having two positions, one called locked, wherein the measuring rod (22) is coupled with the mobile section (86), which allows measuring in static mode at least the cone resistance (qc) whatever the static or dynamic drive-in mode, and the other called free, wherein the measuring rod (22) is uncoupled from said mobile section (86).

2. Device (1) according to claim 1, **characterized in that** said measuring means comprise at least one pressure sensor (83) associated with a gauge (82).

3. Device (1) according to claim 2, **characterized in that** said pressure sensor (83) is hydraulic and **in that** said gauge (82) comprises a compression chamber (82) delimited between a fixed section (84) and a mobile section (86) of said mounting block (8), said mobile section (86) being arranged to be coupled to said measuring rod (22) by said locking means (9).

4. Device (1) according to claim 3, **characterized in that** said locking means (9) comprise at least one lock (90) coupled to an actuator (91) in order to be mobile in a direction perpendicular to the central shaft (30) between a free position wherein said measuring rod (22) is uncoupled from said measuring means and a locked position wherein said measuring rod (22) is coupled to said measuring means.

5. Device (1) according to claim 4, **characterized in that** said central shaft (30) comprises a through axial slot (36) to allow the displacement of said lock (90) in locked position and **in that** said axial recess opens into this axial slot (36) to receive an extension piece (20) of said measuring rod (22).

6. Device (1) according to claim 4, **characterized in that** an indicator (93) is arranged opposite to said actuator (91) so as to be visible from the outside of said mounting block (8) by an operator, said indicator (93) cooperating with said lock (90) to be in a retracted position when the lock (90) is in its free position and in a protruding position when the lock (90) is in its locked position.

7. Device (1) according to any of the previous claims, **characterized in that** said central shaft (30) is slidingly mounted, with a controlled stroke, in said mounting block (8).

8. Device (1) according to any of the previous claims, **characterized in that** the lower end of said central shaft (30) comprises a joining piece (31) to receive said protective tube (32).

9. Device (1) according to claim 8, **characterized in that** it comprises a drive-in guide (33) arranged to be fastened on said joining piece (31) of the central shaft (30), this guide being provided with a housing arranged to cover a drive-in head (34) mounted on said protective tube (32).

10. Device (1) according to claim 8, **characterized in that** it comprises a pull-out head (37) arranged to be fastened on said joining piece (31) of the central shaft (30), this head being provided with a housing arranged to cover a pull-out part (38) fastened on said protective tube (32), and **in that** it comprises a pull-out fork (39) arranged to fit into said pull-out part (38) and make said protective tube (32) integral with said central shaft (30).
